(19) Europäisches Patentamt
European Patent Office
Office européen des brevets

(11) EP 4 171 582 B1

(12) # EUROPEAN PATENT SPECIFICATION

(45) Date of publication and mention of the grant of the patent:
**13.03.2024 Bulletin 2024/11**

(21) Application number: **21847670.3**

(22) Date of filing: **13.09.2021**

(51) International Patent Classification (IPC):
***A61K 31/74*** *(2006.01)* ***C08F 8/12*** *(2006.01)*
***C08F 220/22*** *(2006.01)* ***C08F 220/18*** *(2006.01)*

(52) Cooperative Patent Classification (CPC):
(C-Sets available)
**A61P 3/02; A61K 31/765; A61K 31/78; A61K 31/787; A61K 31/795; A61K 31/80; A61P 3/12; C08F 8/12; C08F 220/22** (Cont.)

(86) International application number:
**PCT/CN2021/118053**

(87) International publication number:
**WO 2023/035274 (16.03.2023 Gazette 2023/11)**

(54) **POLYMER MEDICAMENT FOR TREATING HYPERKALEMIA AND PREPARATION METHOD THEREOF**

POLYMERMEDIKAMENT ZUR BEHANDLUNG VON HYPERKALÄMIE UND HERSTELLUNGSVERFAHREN DAFÜR

MÉDICAMENT POLYMÈRE POUR LE TRAITEMENT DE L'HYPERKALIÉMIE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.05.2023 Bulletin 2023/18**

(73) Proprietor: **Waterstone Pharmaceuticals (Wuhan) Co., Ltd.**
**Wuhan, Hubei 430075 (CN)**

(72) Inventors:
- **FU, Min**
  **Wuhan, Hubei 430075 (CN)**
- **TANG, Huiling**
  **Wuhan, Hubei 430075 (CN)**
- **LI, Tongtong**
  **Wuhan, Hubei 430075 (CN)**
- **HU, Minglong**
  **Wuhan, Hubei 430075 (CN)**
- **BIE, Kang**
  **Wuhan, Hubei 430075 (CN)**
- **LIANG, Ying**
  **Wuhan, Hubei 430075 (CN)**
- **CUI, Jian**
  **Wuhan, Hubei 430075 (CN)**
- **ZHANG, Faming**
  **Wuhan, Hubei 430075 (CN)**

(74) Representative: **Habermann, Hruschka & Schnabel**
**Patentanwälte**
**Montgelasstraße 2**
**81679 München (DE)**

(56) References cited:
**WO-A1-2010/132662**
**WO-A1-2014/015240**
**WO-A1-2017/109658**
**WO-A1-2019/220450**
**CN-A- 102 202 670**
**CN-A- 102 844 334**
**US-A1- 2011 236 340**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08F 8/12, C08F 220/22;**
C08F 220/22, C08F 212/36;
C08F 220/22, C08F 216/125;
C08F 220/22, C08F 226/06;
C08F 220/22, C08F 230/085;
C08F 220/22, C08F 236/20;
C08F 220/22, C08F 236/22

## Description

### FIELD

The present disclosure relates to the field of medicinal chemistry, and particularly, the present disclosure relates to a polymer medicament for treating hyperkalemia and a preparation method thereof.

### BACKGROUND

Potassium ($K^+$) is the most abundant cation in cells, and the content thereof in the human body is about 35 mEq/kg to 40 mEq/kg. Serum potassium in a range of about 5.0 mEq/L to 6.0 mEq/L can be defined as mild hyperkalemia, which is usually not life threatening. However, the moderate to severe hyperkalemia (serum potassium greater than about 6.1 mEq/L) may cause serious consequences. Arrhythmia and ECG waveform distortion are both the characteristics of hyperkalemia. When the serum potassium level rises to about 9 mEq/L or higher, symptoms such as atrioventricular dissociation, ventricular tachycardia, or ventricular fibrillation may occur.

Hyperkalemia is rare in the general healthy population. However, for some populations, hyperkalemia has a higher incidence. Among hospitalized patients, the incidence of hyperkalemia is about 1% to 10%, depending on the definition of hyperkalemia. Severe patients, premature babies or the elderly all belong to the groups at higher risk. Decreased kidney function, genitourinary diseases, cancer, severe diabetes, and mixed administration may all increase the risk of hyperkalemia in patients.

Most of the existing therapeutic regimens for hyperkalemia are limited to hospitalization. Ion-exchange resins, such as Kayexalate, are not suitable for outpatients or long-term treatment due to the fact that large doses must be used and the patients are reluctant to cooperate. Such a treatment has serious side effects on the gastrointestinal (GI) tract and may introduce excess sodium, which potentially leads to hypernatremia, related fluid retention and hypertension. Diuretics can allow patients to eliminate sodium and potassium through the kidneys. Nevertheless, due to the existing nephropathy and related diuretic resistance, the efficacy of diuretics is often limited. In addition, the diuretics are contraindicated in those patients who do not want to have a drop in blood pressure and volume. For example, CHF patients have hypotension, and are often administered with a combination of drugs, using ACE inhibitors and non-potassium diuretics that may induce hyperkalemia, such as spironolactone. US 2011/236340 A1 discloses a pharmaceutical composition that comprises a salt of a crosslinked cation exchange polymer and the use of said polymers for the treatment of hypekalemia.

WO 2014/015240 A1 discloses compositions comprising crosslinked cation-binding polymer comprising monomers containing carboxylic acid groups and pKa decreasing groups used in the treatment of hyperkalemia.

Therefore, it is urgent to develop a new drug with higher binding capacity for treating hyperkalemia.

### SUMMARY

In an aspect of the present disclosure, the present disclosure provides a polymer, the invention being defined by the appending claims. According to an embodiment of the present disclosure, the polymer includes repeating units obtained by polymerizing a monomer and a crosslinking agent. A molar ratio of the monomer to the crosslinking agent ranges from 1:0.02 to 1:0.20. The monomer includes an acidic group and a pKa-reducing group next to the acidic group. The acidic group is selected from the group consisting of sulfonic acid group ($-SO_3^-$), sulfuric acid group ($-OSO_3^-$), carboxylic group ($-CO_2^-$), phosphonic acid group ($-OPO_3^{2-}$), phosphate group ($-OPO_3^{2-}$), and sulfamic acid group ($-NHSO_3^-$). The pKa-reducing group is selected from the group consisting of nitro, cyano, carbonyl, trifluoromethyl, and halogen atoms. The crosslinking agent has three or four reaction sites. Applicant found that the polymer according to the embodiment of the present disclosure has extremely high stability and potassium ion adsorption capacity when being in an acid state rather than a salt state. The polymer according to the embodiment of the present disclosure can be used as a medicament for the effective treatment of hyperkalemia.

According to an embodiment of the present disclosure, the above-mentioned polymer may further include at least one of the following technical features.

According to an embodiment of the present disclosure, the acidic group is the carboxylic group, and the pKa-reducing group is fluorine.

According to an embodiment of the present disclosure, the reaction sites are free alkenyl groups.

According to an embodiment of the present disclosure, the crosslinking agent includes at least one of triallyl isocyanurate, 1,2,4-trivinylcyclohexane, tetravinylsilane, trimethylolpropane trimethacrylate, triallyl cyanurate, or triallylcarbinol.

According to an embodiment of the present disclosure, the polymer is at least one selected from the group consisting of polyvinyl sulfonic acid polymer, polyvinyl sulfamic acid polymer, poly(vinyl sulfamic acid/vinyl sulfuric acid)

copolymer, polyvinyl amino phosphonic acid polymer, N-(bisphosphonate ethyl) polyvinylamine polymer, poly(α-fluoroacrylic acid) polymer, vinylphosphonic acid/acrylic acid copolymer, vinylphosphonic acid/α-fluoroacrylic acid copolymer, polyvinylsulfuric acid polymer, and cross-linked polyvinylsulfamic acid polymer.

In another aspect of the present disclosure, the present disclosure further provides a polymer. According to an embodiment of the present disclosure, the polymer has a structure represented by formula (I) or is a salt of the structure represented by formula (I):

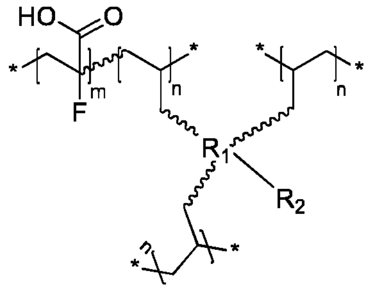

(I),

in which, $R_1$ is

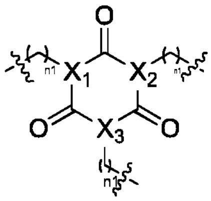
, 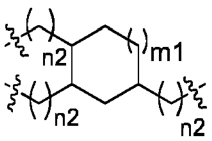
, 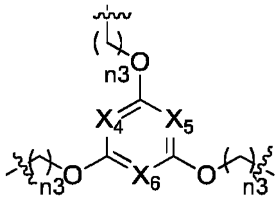
, 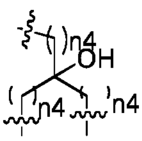
,

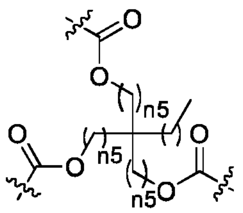
, or 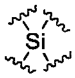
;

when $R_1$ contains three binding sites, $R_2$ is not present; and when $R_1$ contains four binding sites, $R_2$ is connected to one of the four binding sites of $R_1$ and $R_2$ is

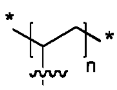
;

m ranges from 0.80 to 0.98, n ranges from 0.02 to 0.20, and m+n=1;
n1, n2, n3, n4, and n5 are each independently selected from 0, 1, 2, 3, 4, 5, 6, or 7;
$X_1$, $X_2$, $X_3$, $X_4$, $X_5$, and $X_6$ are each independently a carbon atom or a nitrogen atom;
m1 is 0 or 1; and
* represents a binding site.

According to an embodiment of the present disclosure, the above-mentioned polymer may further include at least one of the following technical features.

According to an embodiment of the present disclosure, $R_1$ is

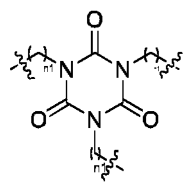

,

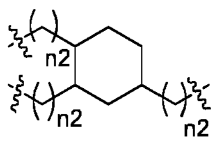

, 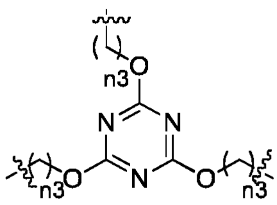

, 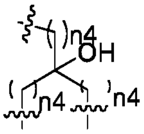

, 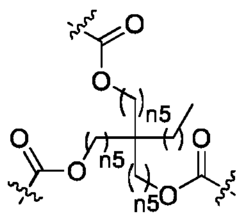

, or 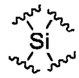

.

According to an embodiment of the present disclosure, $R_1$ is

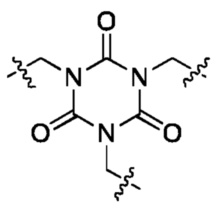

,

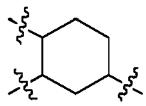

, 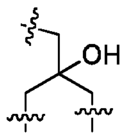

, 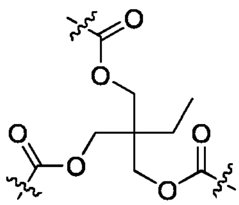

, or 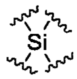

.

In yet another aspect of the present disclosure, the present disclosure further provides a polymer. According to an embodiment of the present disclosure, the polymer has any one of the following structures or is a salt of any one of the following structures:

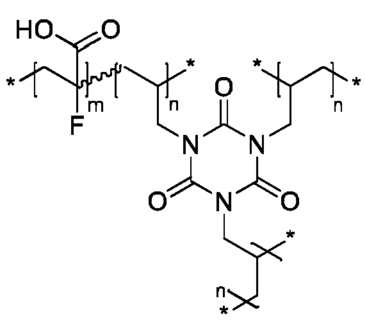

, 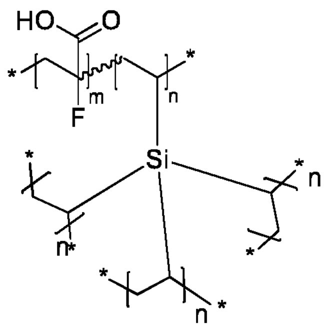

, or 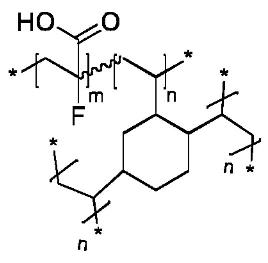

,

in which m ranges from 0.80 to 0.98; n ranges from 0.02 to 0.20; and m+n=1.

In yet another aspect of the present disclosure, the present disclosure further provides a pharmaceutical composition. According to an embodiment of the present disclosure, the pharmaceutical composition includes the polymer as described above, and a pharmaceutically acceptable excipient.

In yet another aspect of the present disclosure, the present disclosure further provides a use of the polymer as described above or the composition as described above in manufacture of a medicament for adsorbing potassium.

In yet another aspect of the present disclosure, the present disclosure further provides a use of the polymer

as described above or the composition as described above in manufacture of a medicament for treating or preventing hyperkalemia.

According to an embodiment of the present disclosure, the above-mentioned use may further include at least one of the following technical features.

According to an embodiment of the present disclosure, the hyperkalemia is caused by administration of a medicament that causes potassium retention.

According to an embodiment of the present disclosure, the medicament that causes potassium retention is selected from the group consisting of non-potassium diuretics, angiotensin-converting enzyme inhibitors, non-steroidal anti-inflammatory drugs, heparin, and trimethoprim.

According to an embodiment of the present disclosure, the present disclosure further provides a method for preparing the above-mentioned polymer. According to an embodiment of the present disclosure, the method includes: adding a monomer, a crosslinking agent, an initiator, a dispersant, and an inorganic salt to water, dissolving and dispersing them uniformly at room temperature, reacting for a period of time at a certain temperature to obtain a solid of the polymer, then removing an alkyl moiety from the polymer to generate a polymer carboxylate, and finally acidifying the polymer carboxylate with an acidic solution to obtain the finished product.

A ratio of the monomer to the crosslinking agent ranges from 1: 0.02 to 1: 0.20, and when the ratio is 1: 0.026, the sample has a higher adsorption capacity for potassium ions.

In some embodiments, both a water-soluble free radical initiator and an oil-soluble free radical initiator can be used to initiate the reaction to obtain the corresponding polymer. The water-soluble initiator includes, but is not limited to, potassium persulfate, ammonium persulfate, 2,2'-azobis(2-methylpropionamidine)dihydrochloride (V50), 2,2'-azabis(2-imidazoline) dihydrochloride (VA044), etc.

The above-mentioned oil-soluble initiator includes, but is not limited to, 2,2'-azobis(2-methylpropionitrile), 2,2'-azobis-(2,4-dimethylvaleronitrile), 2,2-azodi(2-methylbutyronitrile), 1,1'-azobis(cyclohexane-1-carbonitrile), dimethyl 2,2'-azobis(2-methylpropionate), dibenzoyl peroxide, lauroyl peroxide, cumene hydroperoxide, etc.

The dispersant used in certain embodiments includes, but is not limited to, gelatin, polyvinyl alcohol, sodium carboxymethyl cellulose, hydroxymethyl cellulose, sodium polyacrylate, calcium carbonate, magnesium carbonate, barium sulfate, diatomite, Talc powder, Tween 20, Tween 40, Tween 80, Tween 85, Span 20, Span 40, Span 60, Span 65, Span 80, Span 85, etc.

The inorganic salt used in certain embodiments includes, but is not limited to, potassium chloride, sodium chloride, ammonium chloride, calcium chloride, magnesium chloride, etc.

A method for removing alkyl group from the polymer includes hydrolysis using an alkali, and the used alkali includes, but is not limited to, potassium hydroxide, sodium hydroxide, lithium hydroxide, magnesium hydroxide, potassium carbonate, sodium carbonate, etc.

The hydrolysis of the present disclosure is carried out in a mixed solution of an aqueous alkali solution and an organic solvent. The used organic solvent includes, but is not limited to, ethanol, methanol, isopropanol, toluene, acetonitrile, diethyl ether, tetrahydrofuran, etc. It is surprisingly found that during removing of the alkyl moiety using ether solvents, the polymer product prepared with tetrahydrofuran and other ethers has better properties and colors. The ether used includes, but is not limited to, tetrahydrofuran, methyl tert-butyl ether, dimethoxyethane, ethylene glycol diethyl ether, etc.

The acid used in the above acidification of the polymer includes, but is not limited to, sulfuric acid, hydrochloric acid, nitric acid, etc.

In yet another aspect of the present disclosure, the present disclosure further provides a potassium ion adsorption determination method. According to an embodiment of the present disclosure, the method includes: detecting an adsorption capacity of the polymer as described above for potassium ions by ion chromatography.

According to an embodiment of the present disclosure, the conditions of the ion chromatography are as below:

Chromatographic column: IonPac CS17 Analytical Column (4 × 250 mm)
Protection column: IonPac CG17 Guard Column (4 × 50 mm)
Flow rate: 1.0 ml/min
Detector: Electrical conductivity detector
Column temperature: 30°C
Injection volume: 10μl
Eluent: 6mM methanesulfonic acid solution
Running time: 20min.

**Definition and explanation**

Those skilled in the art can understand that the *binding site can be further connected to monomers or the

same or different crosslinking agents.

The present disclosure provides a method, a composition and a kit for treating animals. As used herein, "test animals" and "animals" include humans and other mammals. The present disclosure specifically provides a polymer composition for eliminating potassium ions. Preferably, the composition can be used to eliminate potassium ions from the gastrointestinal tract of animals.

A part of the present disclosure relates to a method for eliminating potassium using a potassium-binding polymer composition. In a certain embodiment, the potassium-binding polymer composition has high potassium-binding energy and/or selectivity, and can release the unbound potassium in the gastrointestinal tract. Preferably, the potassium-binding polymer composition will not release the bound potassium in the rectum. The polymer composition of the present disclosure has binding selectivity for potassium ions.

Preferably, the polymer composition of the present disclosure has a high binding energy and/or selectivity to potassium. The "high binding energy" herein means that each gram of the polymer can bind about 1.5 mmol or more of potassium in the body on average, and the *in vivo* potassium-binding energy herein is usually determined *in vivo.* Techniques for measuring the *in vivo* potassium-binding energy are known. For example, after a patient is given a potassium-binding polymer, the content of potassium in the patient's faeces can be used to calculate the *in vivo* potassium-binding energy. Optimally, an average potassium-binding energy in the human body is obtained through experiments and calculations based on a group of normal subjects. The group of subjects includes about 5 members, preferably about 10 members, more preferably about 25 members, and most preferably about 50 members.

The potassium-binding energy of the polymers of the present disclosure can also be determined *in vitro.* Preferably, the *in vitro* determination of the potassium-binding energy of the polymer of the present disclosure is carried out under the physiological conditions simulating gastrointestinal tract, especially the colon. In certain embodiments, the *in vitro* determination of the potassium-binding energy of the polymers of the present disclosure is performed in a solution having a pH of about 5.5 or higher. In various embodiments, the potassium-binding energy determined in a solution having a pH of about 5.5 or higher is equal to or greater than 6 mmol/g. Preferably, the *in vitro* potassium-binding energy of the polymer, which is determined in a solution having a pH of about 5.5 or higher, is between about 6 mmol/g and about 12 mmol/g. More preferably, the *in vitro* potassium-binding energy of the polymer, which is determined in a solution having a pH of about 5.5 or higher, is equal to or greater than about 6 mmol/g. More preferably, the *in vitro* potassium-binding energy of the polymer is equal to or greater than about 8 mmol/g. More preferably, the *in vitro* potassium-binding energy of the polymer is equal to or greater than about 10 mmol/g. Most preferably, the *in vitro* potassium-binding energy of the polymer is equal to or greater than about 12 mmol/g.

If the polymer composition has high potassium-binding energy, the dose of the composition can be reduced. Generally, the effective therapeutic and preventive dose of the polymer composition ranges from about 0.5 g/day and about 25 g/day. The most preferred dose is equal to or less than about 15 g/day. A preferred dose range is between about 5 g/day to about 20 g/day. A more preferred dose range is between about 5 g/day to about 15 g/day. A more preferred dose range is between about 10 g/day to about 20 g/day. The most preferred dose range is between about 10 g/day to about 15 g/day. Preferably, such a dose is taken before meal three times a day, most preferably once a day.

The polymer composition of the present disclosure can retain a large amount of bound potassium. The polymer binds potassium in the colon and does not release the bound potassium before the polymer is excreted in the faeces. The "large amount" herein does not indicate a capability of retaining all the bound potassium. Preferably, at least a part of the bound potassium is retained in order to achieve the therapeutic and/or preventive effects. It is desirable to retain about 5% to about 100% of the bound potassium. Preferably, the polymer composition can retain about 25% of the bound potassium. More preferably, about 50% of the bound potassium can be retained. More preferably, about 75% of the bound potassium can be retained. Most preferably, about 100% of the bound potassium can be retained. Optimally, a retaining period of the bound potassium is a time period for treatment and/or prevention with the polymer composition. In this embodiment, the polymer composition is used to bind and eliminate potassium in the gastrointestinal tract, and the retention period thereof is a retention period of the composition in the gastrointestinal tract, and preferably an average retention time in the colon.

The potassium-binding polymer is preferably not absorbed by the gastrointestinal tract. The expression "not absorbed by" and its grammatical synonyms do not mean that none of the administered polymer is not absorbed. It is desired that a certain amount of the polymer will not be absorbed. Preferably, about 90% or more of the polymer is not absorbed. More preferably, about 95% or more of the polymer is not absorbed. More preferably, about 97% or more of the polymer is not absorbed. Most preferably, about 98% or more of the polymer is not absorbed.

In some embodiments, the potassium-binding polymer contains various protic or ionic acidic groups, for example, sulfonic acid group ($-SO_3^-$), sulfuric acid group ($-OSO_3^-$), carboxylic group ($-CO_2^-$), phosphonic acid group ($-OPO_3^{2-}$), phosphate group ($-OPO_3^{2-}$), and sulfamic acid group ($-NHSO_3^-$). Optimally, under the physiological pH of colon, an ionization percentage of the acidic groups is greater than 75%, and the potassium-binding energy is greater than 5 mmol/g. Preferably, the ionization percentage of the acidic groups is greater than 80%, more preferably, greater than 90%, and most preferably, approximately equal to 100%.

In certain embodiments, the acid group-containing polymer contains more than one kind of acid groups. In some embodiments, the acidic group-containing polymer taken is actually an acid anhydride-based polymer, which will form an ionic polymer once it comes into contact with physiological fluids. In some other embodiments, the polymer preferably has a pKa-reducing group next to the acidic group, more preferably an electron-withdrawing substituent group, and most preferably, the pKa-reducing group is positioned at $\alpha$ or $\beta$ position of the acidic group. Preferably, the electron-withdrawing substituent group includes hydroxyl, ether groups, ester groups, or halogen atoms, and most preferably fluorine. Preferably, the acidic group is sulfonic acid group ($-SO_3^-$), sulfuric acid group ($-OSO_3^-$), carboxylic group ($-CO_2^-$), phosphonic acid group ($-OPO_3^{2-}$), phosphate group ($-OPO_3^{2-}$), or sulfamic acid group ($-NHSO_3^-$). It is also preferred that the polymer can be prepared through a polymerization reaction of $\alpha$-fluoroacrylic acid, difluoromaleic acid, or corresponding anhydrides thereof.

Suitable phosphonic acid monomers include vinylphosphonic acid, ethylene-1,1-bisphosphonic acid, ethylene derivative of phosphonic acid carboxylates, oligo(methylene phosphonic acid), and hydroxyethane-1,1-bisphosphonic acid. The synthesis methods of these monomers are known.

The free radical polymer prepared from monomers such as vinyl sulfonate, vinyl phosphonate, or vinyl sulfamate can be used.

The preferred monomers used herein are $\alpha$-fluoroacrylate and difluoromaleic acid, and most preferably $\alpha$-fluoroacrylate. These monomers can usually be prepared by a variety of methods (Gassenetal, J. Fluorine Chemistry, 55, (1991) 149-162, KF Pittman, C.U., M. Ueda, etal, Macromolecules (1980), 13(5), 1031-1036). Difluoromaleic acid is preferably prepared by oxidation of fluoroaromatic compounds (Bogachevetal, Zhurnal Organisheskoi Khimii, 1986, 22(12), 2578-83), or by oxidation of fluoro-substituted furan derivatives (US Patent No. 5,112,993). A preferred method for synthesizing $\alpha$-fluoroacrylate has been disclosed in European Patent EP415214.

Other methods include step-growth polymerization of functional compounds, and the functional compound contains a phosphonic acid group, a carboxylic group, a phosphate group, a sulfinic acid group, a sulfate group, or a sulfonic acid group. For example, the high-density polyphosphonic acid sold by Rhodia (Trademark: Briquest) is very suitable for use in the present disclosure.

The polymer of the present disclosure also includes an ion exchange resin synthesized from natural polymers, and the natural polymers may be glycopolymers and semi-synthetic polymers. These polymers can be treated to have ion exchange positions on their backbone or pendant groups. For example, the glycopolymers may be materials derived from plants or animals, including cellulosic materials, hemicellulose, alkyl cellulose, hydroxyalkyl cellulose, carboxymethyl cellulose, ethyl cellulose sulfonate, starch, xylan, amylopectin, chondroitin, hyaluronate ester, heparin, guar gum, xanthan gum, mannan, galactomannan, chitin, and polyglucosamine. The most preferred polymers should not be decomposed or absorbed under the physiological conditions of the gastrointestinal tract, for example, carboxymethyl cellulose, polyglucosamine, and ethyl cellulose sulfonate.

The potassium-binding polymer can be encapsulated in a dialysis bag, paper bag, microporous matrix, polymer gel, hollow fiber, vesicle, capsule, tablet, or membrane.

The polymer can be prepared by homogeneous or heterogeneous polymerization. In the homogeneous reaction, the soluble polymer chain reacts with the cross-linking agent to obtain a cross-linked gel, and further, the cross-linked gel is extruded to form particles or pulverized into smaller particles. In the heterogeneous reaction, particles are obtained through emulsification or dispersion of soluble polymer precursors and then crosslinking. In another method, the particles are prepared by polymerizing monomers in an emulsification, suspension, microemulsification or dispersion process. The continuous phase can be an aqueous solvent or an organic solvent. When adopting the suspension process, any suitable alternative process can be used, including methods such as "template polymerization" and "multistage seeded suspension". Almost all the particles produced by these methods are monodisperse particles. In a particular embodiment, the particles are prepared using a "spraying" process (see US Patent 4,427,794). In this process, a mixture of monomer and initiator is injected into a continuous phase through vibrating nozzles. The nozzles are arranged as a rapidly rotating turntable, and thus the liquid can be accelerated under centrifugal force.

Direct suspension polymerization is one preferred process for the production of $\alpha$-fluoroacrylate particles. Generally, in order to prevent aggregation of particles during the production process, a suspension stabilizer is used, for example, gelatin, polyvinyl alcohol, and sodium polyacrylate, sodium hydroxymethylcellulose. It has been found that the aggregation and particle agglomeration can be reduced by adding NaCl to the aqueous phase. Other salts suitable for this purpose include various salts capable of being dissolved in the aqueous phase. In this embodiment, the added amount of the salt soluble in water ranges from about 0.1% by weight to about 10% by weight, more preferably from about 2% by weight to about 5% by weight, and most preferably from about 3% by weight to about 4% by weight.

Currently, it has been found that during the suspension polymerization of $\alpha$-fluoroacrylate (such as MeFA), the characteristics of the free radical initiator have a greater impact on the quality of the suspension, including particle stability, particle yield, and retention of spherical shape, and other aspects. The use of water-insoluble free radical initiators, such as dodecyl peroxide, will result a high yield. It has been confirmed that the optimal effect will be achieved when the water solubility of the free radical initiator is less than 0.1 g/L, more preferably less than 0.01 g/L. In certain

preferred embodiments, the poly-MeFA particles are generated in presence of the free radical initiator with low water solubility as well as the salt in the aqueous phase, such as NaCl.

## BRIEF DESCRIPTION OF DRAWINGS

The above and/or additional aspects and advantages of the present disclosure will become apparent and easy to understand from the description of embodiments in conjunction with the following drawing:

FIG. 1 is a diagram showing that Sodium zirconium cyclosilicate and MFA-TAIC polymer could reduce the increase of urine $K^+$ induced by KCl.
FIG.2 is a diagram showing that Sodium zirconium cyclosilicate and MFA-TAIC polymer significantly increased fecal $K^+$ excretion ($P < 0.001$, $P < 0.001$).

## DESCRIPTION OF EMBODIMENTS

The present disclosure is described below with reference to specific embodiments. It should be noted that these embodiments are only descriptive and do not limit the present disclosure in any way.
The following abbreviations are used throughout the present disclosure:
MFA: methyl 2-fluoroacrylate; PVA: polyvinyl alcohol; BPO: benzoyl peroxide; TAIC: triallyl isocyanurate; TVS: tetravinylsilane; DGDE: diethylene glycol divinyl ether; DADMS: diallyldimethylsilane; EGDMA: ethylene glycol dimethacrylate; TMPTMA: trihydroxymethylpropyl trimethacrylate; DPGDA: dipropylene glycol diacrylate; DVB: divinylbenzene; and ODE: octadiene.
A positive drug used in the present disclosure is sodium zirconium cyclosilicate (Lokelma), a commercial product from AstraZenca/AndersonBrecon. Inc., batch No.: LJ2076A.
The crosslinking agents used in the present disclosure are listed in Table 1.

[Table 1]

| No. | Name | Abbreviation | Structural formula |
|---|---|---|---|
| (1) | Triallyl isocyanurate | TAIC | 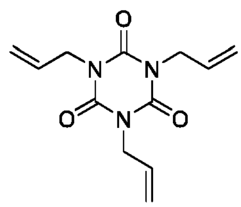 |
| (3) | Tetravinylsilane | TVS | 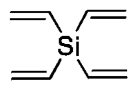 |
| (4) | 1,2,4-trivinylcyclohexane | TVCH | 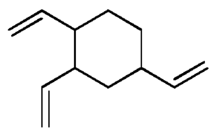 |
| (5) | Diethylene glycol divinyl ether | DGDE | 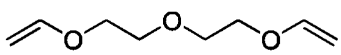 |
| (6) | Diallyldimethylsilane | DADMS | 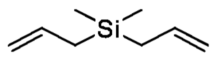 |
| (7) | Ethylene glycol dimethacrylate | EGDMA | 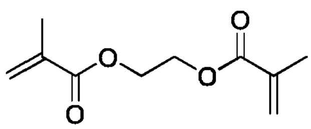 |

(continued)

| No. | Name | Abbreviation | Structural formula |
|---|---|---|---|
| (8) | Trihydroxymethylpropyl trimethacrylate | TMPTMA | 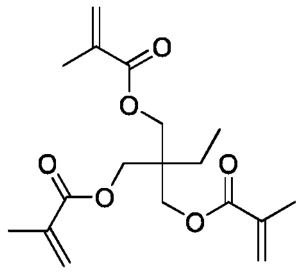 |
| (9) | Dipropylene glycol diacrylate | DPGDA | 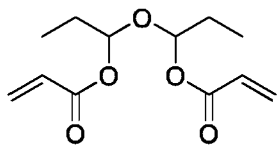 |
| (10) | Divinylbenzene | DVB | 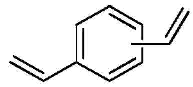 |
| (11) | Octadiene | ODE | 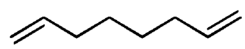 |

Example 1

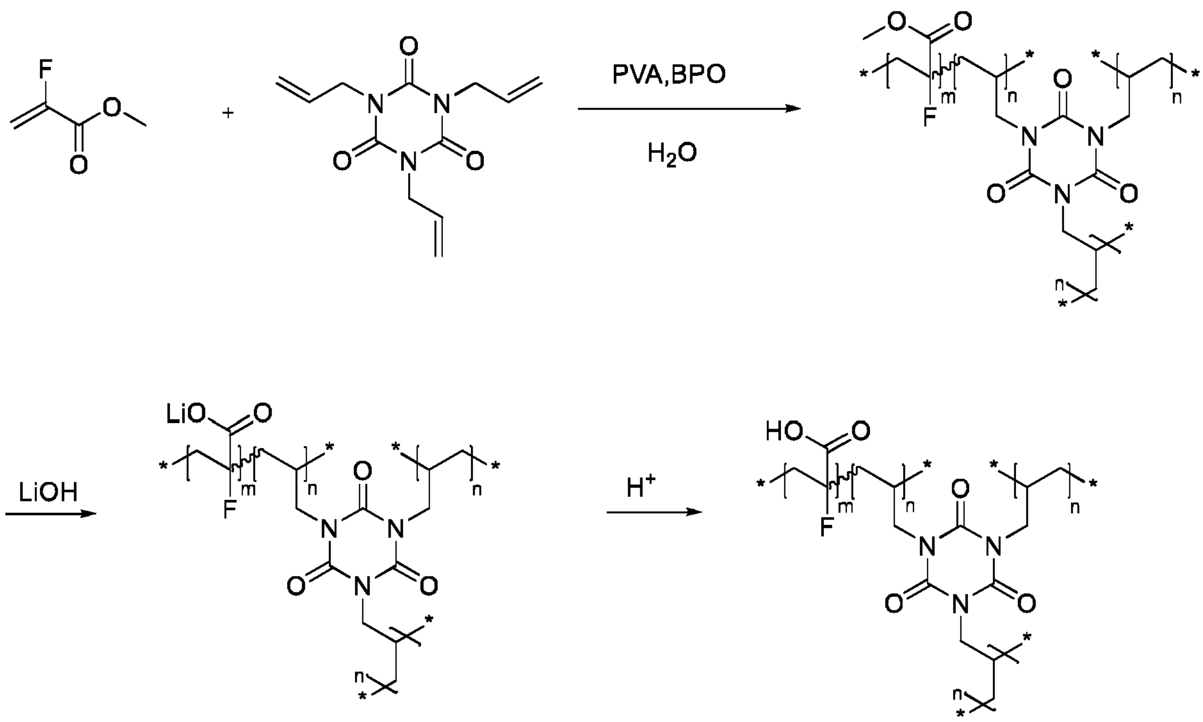


550 mL of purified water and 3.4 g of PVA were added to a reaction flask, and dissolved by stirring at 20°C to 30°C until they were completely dissolved clarification. MFA solution was prepared as below: 104.0 g of MFA, 14.0 g of TAIC, and 0.85 g of BPO were stirred and completely dissolved clarification for later use. The prepared MFA solution was added into the reaction flask. The reaction temperature was gradually increased to 70°C to 80°C, followed by holding the temperature and stirring for 15h. After the temperature was reduced to 20°C to 30°C, suction filtration was performed.

The filter cake was pulped and washed respectively with 1L water and 1L ethanol, 3 times each. The obtained wet product was vacuum-dried at 50°C to obtain 101.0 g of a white solid, i.e., MFA-TAIC polymer ester.

400 mL of water, 50.4 g of lithium hydroxide monohydrate, and 150 mL of tetrahydrofuran were added to a reaction flask, and then the above MFA-TAIC polymer ester was added under stirring. The temperature was increased to 50°C to 60°C, followed by stirring and holding the temperature for 15h. The temperature was lowered to 20°C to 30°C, then filtration was performed, and the filter cake was pulped and washed with water and ethanol. A lithium salt wet product of MFA-TAIC polymer was obtained by the filtration.

500 mL of water and 100 mL of concentrated hydrochloric acid were added to a reaction flask, the above-mentioned lithium salt wet product of MFA-TAIC polymer was added, followed by stirring for 15h at 20°C to 30°C. After the filtration, the filter cake was pulped and washed with water. The wet product obtained after filtration was pulped once with 500 mL of ethanol. The wet product obtained after filtration was vacuum-dried at 50°C for 8h to obtain 91.0 g of white dry product, which was crushed and sieved through a 120-mesh sieve, to obtain the finished product of the MFA-TAIC(m=0.95, n=0.05) polymer .

**Example 2**

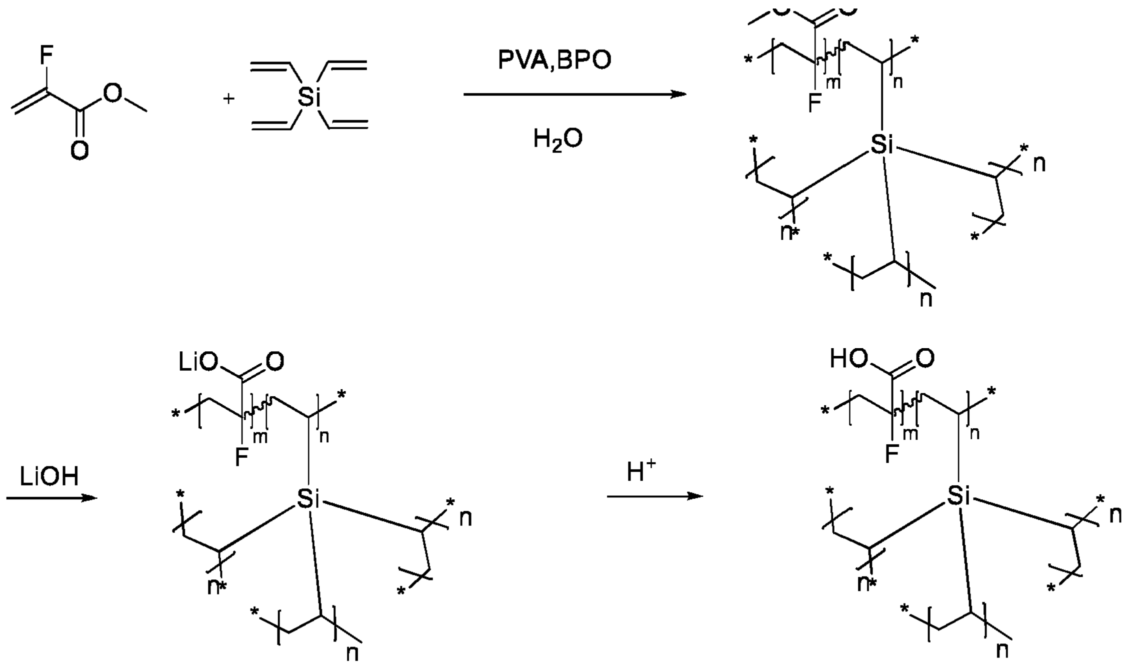


550 mL of purified water and 3.4 g of PVA were added to a reaction flask, and dissolved by stirring at 20°C to 30°C until they were completely dissolved clarification. An MFA solution was prepared as below: 104.0 g of MFA, 6.8 g of TVS, and 0.85 g of BPO were stirred and completely dissolved clarification for later use. The prepared MFA solution was added into the reaction flask. The temperature of the materials in the reaction flask was gradually increased to 70°C to 80°C, followed by holding the temperature and stirring for 15h. After the temperature was reduced to 20°C to 30°C, suction filtration was performed. The filter cake was pulped and washed with water. The obtained wet product was dried to obtain 90.2 g of a white solid, i.e., MFA-TVS polymer ester.

400 mL of water and 50.4 g of lithium hydroxide monohydrate were added to a reaction flask, and then the above MFA-TVS polymer ester was added under stirring. The temperature was increased to 50°C to 60°C, followed by stirring and holding the temperature for 15h. The temperature was lowered to 20°C to 30°C, then filtration was performed, and the filter cake was pulped and washed with water. The filtered wet product was a lithium salt wet product of MFA-TVS polymer.

500 mL of water and 100 mL of concentrated hydrochloric acid were added to a reaction flask, the above-mentioned lithium salt wet product of MFA-TVS polymer was added, followed by stirring for 15h at 20°C to 30°C. After filtration, the filter cake was repeatedly washed with water. The wet product obtained after filtration was pulped once with ethanol. The wet product obtained after filtration was vacuum-dried at 50°C for 8h to obtain 79.4 g of white dry product, which was crushed and sieved through a 120-mesh sieve, to obtain the finished product of the MFA-TVS

(m=0.95, n=0.05) polymer.

Example 3

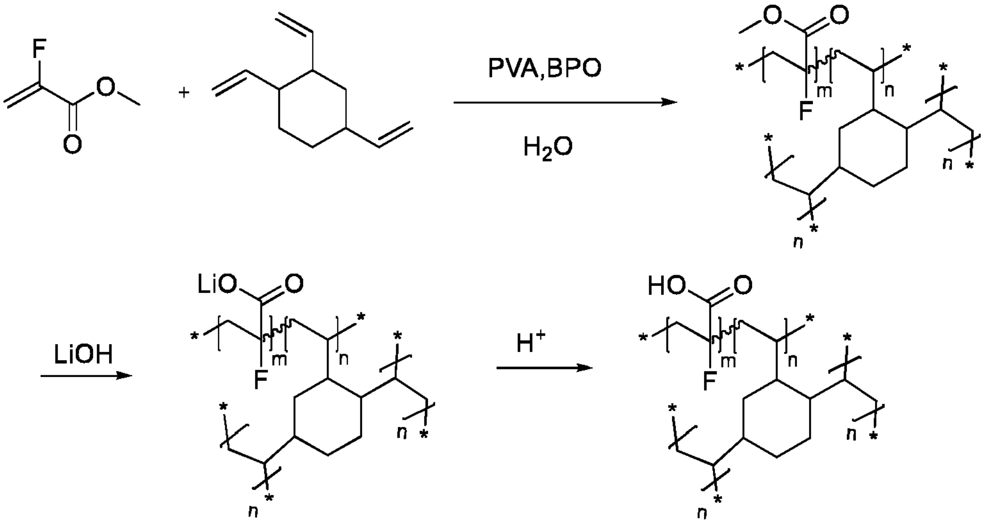


550 mL of purified water and 3.4 g of PVA were added to a reaction flask, and dissolved by stirring at 20°C to 30°C until they were completely dissolved clarification. An MFA solution was prepared as below: 104.0 g of MFA, 8.1 g of TVCH, and 0.85 g of BPO were stirred and completely dissolved clarification for later use. The prepared MFA solution was added into the reaction flask. The temperature of the materials in the reaction flask was gradually increased 70°C to 80°C, followed by holding the temperature and stirring for 15h. After the temperature was reduced to 20°C to 30°C, suction filtration was performed. The filter cake was pulped and washed with water. The obtained wet product was dried to obtain 101.2 g of a white solid, i.e., MFA-TVCH polymer ester.

400 mL of water and 50.4 g of lithium hydroxide monohydrate were added to a reaction flask, and then the above MFA-TVCH polymer ester was added under stirring. The temperature was increased to 50°C to 60°C, followed by stirring and holding the temperature for 15h. The temperature was lowered to 20°C to 30°C, then filtration was performed, and the filter cake was pulped and washed with water. The filtered wet product was a lithium salt wet product of MFA-TVCH polymer.

500 mL of water and 100 mL of concentrated hydrochloric acid were added to a reaction flask, the above-mentioned lithium salt wet product of MFA-TVCH polymer was added, followed by stirring for 15h at 20°C to 30°C. After filtration, the filter cake was repeatedly washed with water. The wet product obtained after filtration was pulped once with ethanol. The wet product obtained after filtration was vacuum-dried at 50°C for 8h to obtain 82.7 g of a white dry product, which was crushed and sieved through a 120-mesh sieve, to obtain the finished product of the MFA-TVCH (m=0.95, n=0.05) polymer.

Comparative Example 4

550 mL of purified water and 3.4 g of PVA were added to a reaction flask, and dissolved by stirring at 20°C to 30°C until they were completely dissolved clarification. An MFA solution was prepared as below: 104.0 g of MFA, 6.5 g of DVB, and 0.85 g of BPO were stirred and completely dissolved clarification for later use. The prepared MFA solution was added into the reaction flask. The temperature of the materials in the reaction flask was gradually increased to 70°C to 80°C, followed by holding the temperature and stirring for 15h. After the temperature was reduced to 20°C to 30°C, suction filtration was performed. The filter cake was pulped and washed with water. The obtained wet product was dried to obtain 112.0 g of a white solid, i.e., MFA-DVB polymer ester.

400 mL of water and 50.4 g of lithium hydroxide monohydrate were added to a reaction flask, and then the above MFA-DVB polymer ester was added under stirring. The temperature was increased to 50°C to 60°C, followed by stirring and holding the temperature for 15h. The temperature was lowered to 20°C to 30°C, then filtration was performed, and the filter cake was pulped and washed with water. The filtered wet product was a lithium salt wet product of MFA-DVB polymer.

500 mL of water and 100 mL of concentrated hydrochloric acid were added to a reaction flask, the above-mentioned lithium salt wet product of MFA-DVB polymer was added, followed by stirring for 15h at 20°C to 30°C. After filtration, the filter cake was repeatedly washed with 4 L of water. The wet product obtained after filtration was pulped once with 500 mL of ethanol. The wet product obtained after filtration was dried at 50°C for 8h to obtain 82.3 g of a white dry product, which was crushed and sieved through a 120-mesh sieve, to obtain the finished product of the MFA-DVB (m=0.95, n=0.05) polymer.

Comparative Example 5

550 mL of purified water and 3.4 g of PVA were added to a reaction flask, and dissolved by stirring at 20°C to 30°C until they were completely dissolved clarification. An MFA solution was prepared as below: 104.0 g of MFA, 5.5 g of ODE, and 0.85 g of BPO were stirred and completely dissolved clarification for later use. The prepared MFA solution was added into the reaction flask. The temperature of the materials in the reaction flask was gradually increased to 70°C to 80°C, followed by holding the temperature and stirring for 15h. After the temperature was reduced to 20°C to 30°C, suction filtration was performed. The filter cake was pulped and washed with water. The obtained wet product was dried to obtain 107.0 g of a white solid, i.e., MFA-ODE polymer ester.

400 mL of water and 50.4 g of lithium hydroxide monohydrate were added to a reaction flask, and then the above MFA-ODE polymer ester was added under stirring. The temperature was increased to 50°C to 60°C, followed by stirring and holding the temperature for 15h. The temperature was lowered to 20°C to 30°C, then filtration was performed, and the filter cake was pulped and washed with water. The filtered wet product was a lithium salt wet product of MFA-ODE polymer.

500 mL of water and 100 mL of concentrated hydrochloric acid were added to a reaction flask, the above-mentioned lithium salt wet product of MFA-ODE polymer was added, followed by stirring for 15h at 20°C to 30°C. After filtration, the filter cake was repeatedly washed with 4 L of water. The wet product obtained after filtration was pulped once with 500 mL of ethanol. The wet product obtained after filtration was dried at 50°C for 8h to obtain 80.3 g of a white dry product, which was crushed and sieved through a 120-mesh sieve, to obtain the finished product of the MFA-ODE (m=0.95, n=0.05) polymer.

**Example 6**

550 mL of purified water and 3.4 g of PVA were added to a reaction flask, and dissolved by stirring at 20°C to 30°C until they were completely dissolved clarification. An MFA solution was prepared as below: 104.0 g of MFA, 14.0 g of TAIC, and 0.85 g of BPO were stirred and completely dissolved clarification for later use. The prepared MFA solution was added into the reaction flask. The temperature of the materials in the reaction flask was gradually increased to 70°C to 80°C, followed by holding the temperature and stirring for 15h. After the temperature was reduced to 20°C to 30°C, suction filtration was performed. The filter cake was pulped and washed with water. The obtained wet product was dried to obtain 101.0 g of a white solid, i.e., MFA-TAIC polymer ester.

400 mL of water and 48.0 g of sodium hydroxide were added to a reaction flask, and then the above MFA-TAIC polymer ester was added under stirring. The temperature was increased to 50°C to 60°C, followed by stirring and holding the temperature for 15h. The temperature was lowered to 20°C to 30°C, then filtration was performed, and the filter cake was pulped and washed with 1 L of water 3 times. The filtered wet product was a sodium salt wet product of MFA-TAIC polymer. The wet product obtained after filtration was dried at 50°C for 12h to obtain 93.7 g of a white dry product, which was crushed and sieved through a 120-mesh sieve, to obtain the finished product of the MFA-TAIC (m=0.95, n=0.05) polymer.

**Example 7**

550 mL of purified water and 3.4 g of PVA were added to a reaction flask, and dissolved by stirring at 20°C to 30°C until they were completely dissolved clarification. An MFA solution was prepared as below: 104.0 g of MFA, 14.0 g of TAIC, and 0.85 g of BPO were stirred and completely dissolved clarification for later use. The prepared MFA solution was added into the reaction flask. The temperature of the materials in the reaction flask was gradually increased to 70°C to 80°C, followed by holding the temperature and stirring for 15h. After the temperature was reduced to 20°C to 30°C, suction filtration was performed. The filter cake was pulped and washed with water. The obtained wet product was dried to obtain 101.0 g of a white solid, i.e., MFA-TAIC polymer ester.

400 mL of water and 48.0 g of sodium hydroxide were added to a reaction flask, and then the above MFA-TAIC polymer ester was added under stirring. The temperature was increased to 50°C to 60°C, followed by stirring and holding the temperature for 15h. The temperature was lowered to 20°C to 30°C, then filtration was performed, and the filter cake was pulped and washed with water. The filtered wet product was a sodium salt wet product of MFA-TAIC polymer.

The filtered wet product was added to 1 L of water, 333.0 g of anhydrous calcium chloride was added, followed

by pulping and stirring at 20°C to 30°C for 6h. The filtered wet product was repeatedly pulped and washed with 1 L water 3 times. The filtered wet product was added with 1 L of water and 333.0 g of anhydrous calcium chloride, followed by pulping and stirring at 20°C to 30°C for 6h. The filtered wet product was repeatedly pulped with 1 L of water until the conductivity was ≤50 μs/cm. After suction filtration, the wet product was dried at 50°C for 18h to obtain 99.2 g of a white dry product, which was crushed and sieved through a 120-mesh sieve, to obtain the finished product of the MFA-TAIC calcium salt (m=0.95, n=0.05) polymer.

**Example 8**

Potassium buffer: potassium buffer was prepared with 200 mmol/L 2-[morpholino] ethanesulfonic acid, the pH was 6.0 to 8.0.

Standard graph: Identify five 100ml volumetric flasks by the numbers 1, 2, 3, 4, and 5. In that order pipet 1, 3, 6. 8, and 10mL of potassium buffer into the flasks, dilute with water to volume, and mix. Perform ion chromatography detection on volumetric flasks 1, 2, 3, 4, and 5 and record the peak area of potassium ion. On ruled coordinate paper, plot the observed peak area as the ordinate, and the concentrations, in mmol per liter, of potassium as the abscissa.

Test sample solution 1: Take about 1.6g of polymer, place it in a 250ml Erlenmeyer flask, add 100ml of potassium buffer, water bath at 37°C±2°C, stir with magnet for 24h, shake evenly, sample (15min, 3h, 5h or 24h as recommended), filter, precisely pipet 1ml of filtrate into a 25ml volumetric flask, and dilute to the mark with water. Test sample solution 1 was prepared for the test of the polymer in an acid form.

Test sample solution 2: Take about 1.6g of polymer salt, exposure to 4 N hydrochloric acid (HCl), washing in water to remove excess HCl. place it in a 250ml Erlenmeyer flask, add 100ml of potassium buffer, water bath at 37°C ±2°C, stir with magnet for 24h, shake evenly, sample (15min, 3h, 5h or 24h as recommended), filter, precisely pipet 1ml of filtrate into a 25ml volumetric flask, and dilute to the mark with water. Test sample solution 2 was prepared for the test of the polymer in a salt form (e.g., metal salt).

Analyze test sample solution 1 and test sample solution 2 by ion chromatography and record the peak area of potassium ion, and determine the potassium concentration, in mmol per liter, by interpolation from the Standard graph. Calculate the adsorption amount, in mmol per g, of potassium ion adsorbed on the resin taken by the formula:

$$\text{Potassium ion adsorption amount of polymer} = (X-2.5Y)/W$$

in which X is the weight, in mmol, of potassium in 100 mL of Potassium solution before exchange; Y is the weight, in mmol, of potassium per L as interpolated from the Standard graph; and W is the weight, in g, of polymer taken, expressed on the anhydrous basis.

The chromatographic conditions are listed in Table 2 below.

[Table 2]

| | |
|---|---|
| Chromatographic column | CS16, CS17, CS12A (250 mm×4 mm) |
| protection column | CG16, CG17, CG12A (50 mm×4 mm) |
| Detector | Electrical conductivity detector |
| Suppressor | CSRS 4 mm |
| Flow rate | 0.3 to 5 ml/min, preferably 1.0 ml/min |
| Column temperature | 30°C |
| Current | 18mA |
| Detector temperature | 35°C |
| Injection volume | 10 to 100 μl, preferably 10 μl |
| Eluent | Methanesulfonic acid solution, preferably 6 mM |
| Running time | 20min |

Results and Analysis

The potassium ion adsorption capacities of the polymers in Examples 1, 2, 3 and Comparative Example 5 were

measured with this method, as shown in Table 3 below.

[Table 3]

| Example No. | Potassium ion adsorption amount of polymer (mmol/g) |
|---|---|
| Example 1 (MFA-TAIC) | 7.5 |
| Example 2 (MFA-TVS) | 7.5 |
| Example 3 (MFA-TVCH) | 7.7 |
| C. Example 5 (MFA-ODE) | 6.7 |
| Sodium zirconium cyclosilicate (commercial product from AstraZenca) | 3.6 |
| Veltassa (commercial product from Relypsa) | 2.6 |

**Conclusion:** It can be seen from the above table that the polymer prepared by using the crosslinking agent with three or four reaction sites in the present disclosure has a significantly higher potassium ion-adsorption capacity than the polymer prepared by using the crosslinking agent with two reaction sites, and significantly higher than the polymers known in the related art.

**Example 9**

The samples listed in Table 4 below were placed in an aluminum plastic bag for Stability test (25°C±2°C/60%RH±5%RH, 40°C±2°C/75%RH±5%RH), and the results are shown in Table 4.

[Table 4]

| Material name | Process | Free fluoride ion content (μg/g) (day 0) | Accelerated stability test (25°C±2°C/60%RH±5%RH) | Accelerated stability test (40°C±2°C/75%RH±5 %RH) |
|---|---|---|---|---|
| Example 1 | MFA-TAIC | 36.0 | Placed at 25°C for 2 months, the free fluoride ion content not increased | Placed at 40°C for 2 months, the free fluoride ion content not increased |
| Veltassa | Commercial product from Relypsa (batch number CCWVX) | 14.3 | Placed at 25°C for 2 months, the free fluoride ion content significantly increased, 8.0 times that on day 0 | Placed at 40°C for 2 months, the free fluoride ion content significantly increased, 25.1 times that on day 0 |

It can be seen from the above table that the polymers of the present disclosure have high stability, which is significantly higher than the polymer of the related art.

**Example 10**

32 normal male SD rats were reared adaptively for 3-5 days and then randomly divided into 4 groups, i.e., a normal group, a model group, a positive control group (sodium zirconium cyclosilicate), a MFA-TAIC polymer group, each group including 8 rats. In accordance with intragastric administration at a volume of 20 ml/kg, the normal group was administered with a corresponding solvent (0.1% xanthan gum), the positive control group was administered with sodium zirconium cyclosilicate at 1.5 g/kg, the MFA-TAIC polymer group was administered with MFA-TAIC polymer at 1.5 g/kg, for only one time. The model group, the positive control group (sodium zirconium cyclosilicate), and the MFA-TAIC polymer group were respectively intraperitoneally injected with 10% KCl at a volume of 4 ml/kg 3 hours after the administration, and then supplemented with 5% KCl solution every 1 hour, continuously for 8 hours, i.e., supplemented with KCl solution 8 times. Before the administration, 3 hours after the administration (i.e., before the modeling (0h)), and 1h, 2h, 4h, 6h, and 8h after the modeling, 0.4 mL of whole blood was collected from the orbit, serum was separated, and blood potassium level was detected. All animals were individually placed in metabolic cages, and urine and faeces within 24 hours after administration were collected on the next day to detect potassium ion content. If the blood collection

and KCl injection had conflicts in term of time, the blood was collected 2 minutes in advance, and then KCl was intraperitoneally injected on time after the blood collection. If deaths occurred, the time and number of deaths were recorded. The faeces were freeze-dried, and the weight of dried faeces of each rat was recorded. Cations in the faeces were extracted with 1M HC1 overnight. Then, the ion chromatography analysis was performed.

The results showed that compared with the normal group, the urine $K^+$ level in the model group was significantly increased ($P < 0.05$), and sodium zirconium cyclosilicate and MFA-TAIC polymer could reduce the increase of urine $K^+$ induced by KCl, as shown in FIG. 1. Compared with normal group, $K^+$ in faeces of model group increased to a certain extent, increasing by 79.87%, with no statistical difference. Compared with the normal group, sodium zirconium cyclosilicate and MFA-TAIC polymer increased fecal $K^+$ excretion ($P < 0.001$, $P < 0.001$), and compared with the model group, sodium zirconium cyclosilicate and MFA-TAIC polymer increased fecal $K^+$ excretion ($P < 0.001$, $P < 0.001$), as shown in FIG. 2.

## Claims

1. A polymer, comprising repeating units obtained by polymerizing a monomer and a crosslinking agent, wherein a molar ratio of the monomer to the crosslinking agent ranges from 1:0.02 to 1:0.20, and the monomer comprises an acidic group and a pKa-reducing group next to the acidic group, wherein the acidic group is selected from the group consisting of a sulfonic acid group ($-SO_3^-$), a sulfuric acid group ($-OSO_3^-$), a carboxylic group ($-CO_2^-$), a phosphonic acid group ($-OPO_3^{2-}$), a phosphate group ($-OPO_3^{2-}$), and a sulfamic acid group ($-NHSO_3^-$); the pKa-reducing group is selected from the group consisting of nitro, cyano, carbonyl, trifluoromethyl, and halogen atoms; and the crosslinking agent has three or four reaction sites.

2. The polymer according to claim 1, wherein the acidic group is the carboxylic group, and the pKa-reducing group is fluorine.

3. The polymer according to claim 1, wherein the reaction sites are free alkenyl groups.

4. The polymer according to claim 1, wherein the crosslinking agent comprises at least one of triallyl isocyanurate, 1,2,4-trivinylcyclohexane, tetravinylsilane, trimethylolpropane trimethacrylate, triallyl cyanurate, or triallylcarbinol.

5. The polymer according to claim 1, wherein the polymer is at least one selected from the group consisting of polyvinyl sulfonic acid polymer, polyvinyl sulfamic acid polymer, poly(vinyl sulfamic acid/vinyl sulfuric acid) copolymer, polyvinyl amino phosphonic acid polymer, N-(bisphosphonate ethyl) polyvinylamine polymer, poly($\alpha$-fluoroacrylic acid) polymer, vinylphosphonic acid/acrylic acid copolymer, vinylphosphonic acid/$\alpha$-fluoroacrylic acid copolymer, polyvinyl sulfuric acid polymer, and cross-linked polyvinylsulfamic acid polymer.

6. A polymer, having a structure represented by formula (I) or being a salt of the structure represented by formula (I):

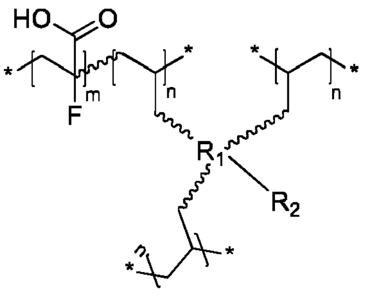

(I),

wherein, $R_1$ is

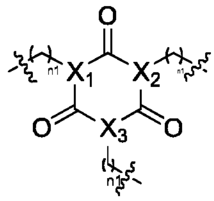, 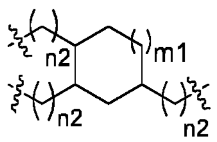, 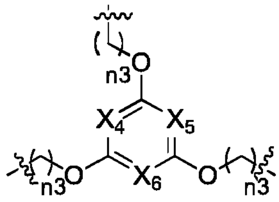, 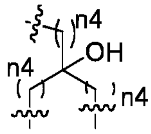,

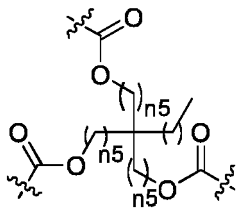, or 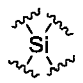;

when $R_1$ contains three binding sites, $R_2$ is not present; and when $R_1$ contains four binding sites, $R_2$ is connected to one of the four binding sites of $R_1$ and $R_2$ is

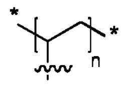;

m ranges from 0.80 to 0.98, n ranges from 0.02 to 0.20, and m+n=1;
n1, n2, n3, n4, and n5 are each independently selected from 0, 1, 2, 3, 4, 5, 6, or 7;
$X_1$, $X_2$, $X_3$, $X_4$, $X_5$, and $X_6$ are each independently a carbon atom or a nitrogen atom;
m1 is 0 or 1; and
* represents a binding site.

**7.** The polymer according to claim 6, wherein $R_1$ is

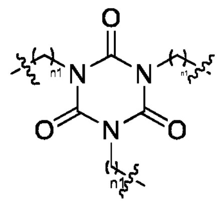, 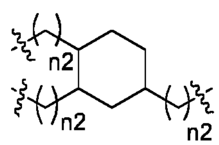,

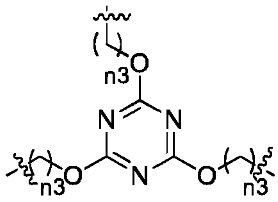, 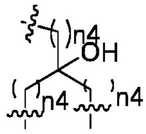, 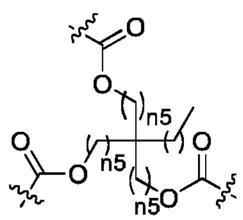, or 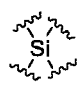.

**8.** The polymer according to claim 6, wherein $R_1$ is

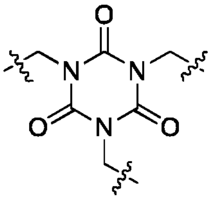, 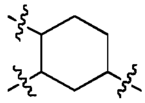,

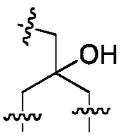, 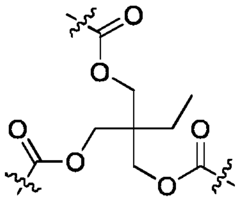, or 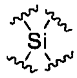.

9. A polymer, having any one of the following structures or being a salt of any one of the following structures:

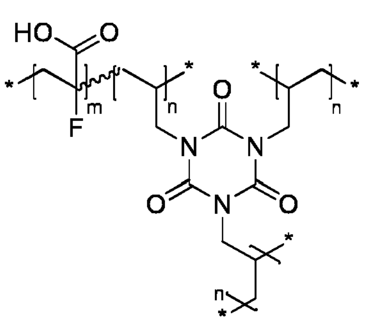, 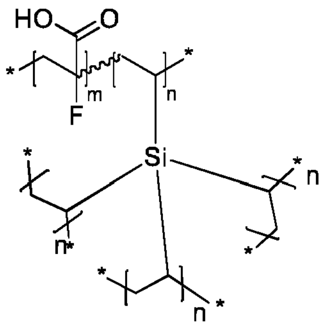, or 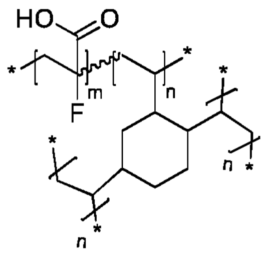,

wherein m ranges from 0.80 to 0.98; n ranges from 0.02 to 0.20; and m+n=1.

10. A pharmaceutical composition, comprising the polymer according to any one of claims 1 to 9, and a pharmaceutically acceptable excipient.

11. The polymer according to any one of claims 1 to 9 or the composition according to claim 10 for use in adsorbption of potassium in a subject.

12. The polymer according to any one of claims 1 to 9 or the composition according to claim 10 for use in prevention or treatment of hyperkalemia.

13. The polymer according to any one of claims 1 to 9 or the composition according to claim 10 for use according to claim 12, wherein the hyperkalemia is caused by administration of a medicament that causes potassium retention.

14. The polymer according to any one of claims 1 to 9 or the composition according to claim 10 for use according to claim 13, wherein the medicament that causes potassium retention is selected from the group consisting of non-potassium diuretics, angiotensin-converting enzyme inhibitors, non-steroidal anti-inflammatory drugs, heparin, and trimethoprim.

15. A potassium ion adsorption determination method, comprising: detecting an adsorption capacity of the polymer according to any one of claims 1 to 9 for potassium ions through ion chromatography.

**Patentansprüche**

1. Polymer mit sich wiederholende Einheiten, erhalten durch Polymerisierung eines Monomers und eines Quervernetzungsmittels, wobei das molare Verhältnis des Monomers zu dem Quervernetzungsmittel im Bereich von 1:0,02 bis 1:0,20 liegt, und das Monomer eine Säuregruppe und ein den pKa-Wert reduzierende Gruppe, die sich neben

der Säuregruppe befindet, umfasst, wobei die Säuregruppe aus der Gruppe, bestehend aus einer Sulfonsäuregruppe ($-SO_3^-$),, einer Schwefelsäuregruppe ($-OSO_3^-$), einer Carbonsäuregruppe ($-CO_2^-$), einer Phosphonsäuregruppe , einer Phosphatgruppe ($-OPO_3^{2-}$) und einer Sulfaminsäuregruppe ($-NHSO_3^-$), ausgewählt ist, die den pKa-Wert reduzierende Gruppe aus der Gruppe, betehend aus Nitro, Cyano, Carbonyl, Triffluormethyl und Halogenatomen, ausgewählt ist, und das Quervernetzungsmittel drei oder vier Reaktionsstellen aufweist.

2. Polymer nach Anspruch 1, wobei die Säuregruppe die Carbonsäuregruppe ist und die den pKa-Wert reduzierende Gruppe Fluor ist.

3. Polymer nach Anspruch 1, wobei die Reaktionsstellen freie Alkenylgruppen sind.

4. Polymer nach Anspruch 1, wobei das Quervernetzungsmittel mindestens eines von Triallylisocyanurate, 1,2,4-Trivinylcyclohexan, Tetravinylsilantrimethylolpropantrimethacrylat, Triallylcyanurat, oder Triallylcarbinol umfasst.

5. Polymer nach Anspruch 1, wobei das Polymer mindestens eines ist, das aus der Gruppe, bestehend aus Polyvinylsulfonsäurepolymer, Polyvinylsulfaminsäurepolymer, Poly(vinylsulfaminsäure/Vinylschwefelsäure)-Copolymer, Polyvinylaminophosphonsäurepolymer, N-(Bisphosphonat-ethyl)-Polyvinylaminpolymer, Poly($\alpha$-Fluoracrylsäure)-Polymer, Vinylphosphonsäure/Acrylsäure-Copolymer, Vinylphosphonsäure/$\alpha$-Fluoracrylsäure-Copolymer, Polyvinylschwefelsäurepolymer und vernetztes Polyvinylsulfaminsäurepolymer.

6. Polymer, das eine Struktur, die in der Formel (I) dargestellt ist, aufweist oder das ein Salz der Struktur ist, die in der Formel (I) dargestellt ist:

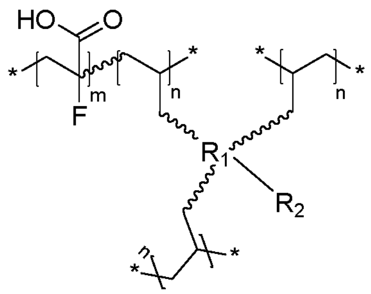

(I),

wobei, $R_1$

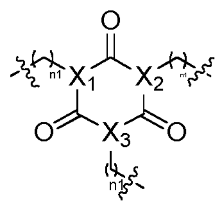
,
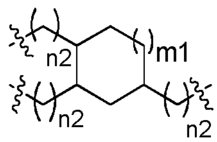
,
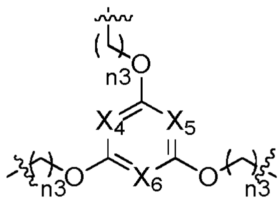
,
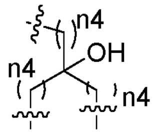
,

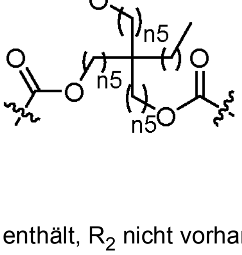
oder
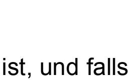

ist;

wobei, falls $R_1$ drei Bindungsstellen enthält, $R_2$ nicht vorhanden ist, und falls $R_1$ vier Bindungsstellen enthält, $R_2$ mit einer der vier Bindungsstellen von $R_1$ verbunden ist und $R_2$

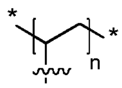

ist,
m im Bereich von 0,80 bis 0,98 liegt, n im Bereich von 0,02 bis 0,20 liegt, und
m+n=1 ist,
n1, n2, n3, n4 und n5 unabhängig voneinander aus 1, 2, 3, 4, 5, 6, oder 7 ausgewählt sind,
$X_1$, $X_2$, $X_3$, $X_4$, $X_5$, und $X_6$ unabhänig voneinander ein Kohlenstoffatom oder ein Stickstoffatom sind,
m1 0 oder 1 ist, und
* eine Bindungsstelle darstellt.

**7.** Polymer nach Anspruch 1, wobei $R_1$

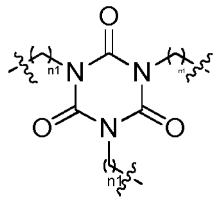 , 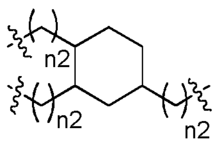 ,

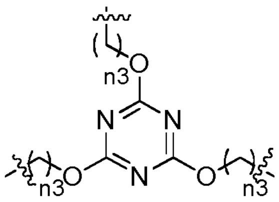 , 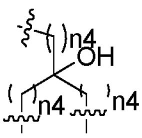 , 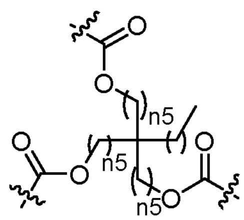 oder 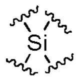 ist.

**8.** Polymer nach Anspruch 6, wobei $R_1$

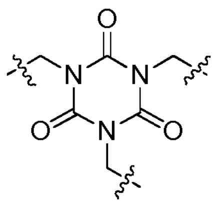 , 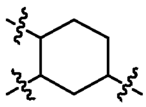 , 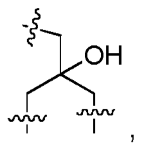 ,

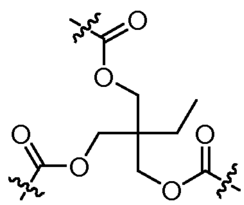 oder 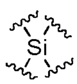

ist.

**9.** Polymer, das eine der folgenden Strukturen aufweist, oder das ein Salz einer der folgenden Strukturen ist:

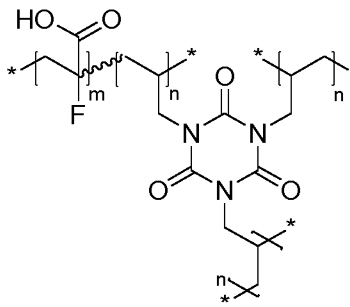 , 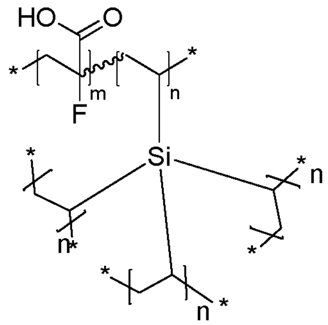 oder 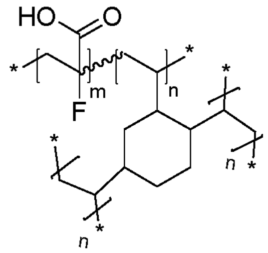 ,

wobei m im Bereich von 0,80 bis 0,98 liegt, n im Bereich von 0,02 bis 0,20 liegt, und m+n=1 ist.

**10.** Pharmazeutische Zusammensetzung, umfassend das Polymer nach einem der Ansprüche 1 bis 9 und einen pharmazeutisch verträglichen Hilfsstoff.

**11.** Polymer nach einem der Ansprüche 1 bis 9 oder Zusammensetzung nach Anspruch 10 zur Verwendung bei der Adsorption von Kalium in einem Subjekt.

**12.** Polymer nach einem der Ansprüche 1 bis 9 oder Zusammensetzung nach Anspruch 10 zur Verwendung bei der Prävention oder Behandlung von Hyperkaliämie.

**13.** Polymer nach einem der Ansprüche 1 bis 9 oder Zusammensetzung nach Anspruch 10 zur Verwendung nach Anspruch 12, wobei die Hyperkaliämie durch Verabreichung eines Medikaments verursacht wird, das eine Kaliumretention verursacht.

**14.** Polymer nach einem der Ansprüche 1 bis 9 oder Zusammensetzung nach Anspruch 10 zur Verwendung nach Anspruch 13, wobei das Medikament, das eine Kaliumretention verursacht, aus der Gruppe, bestehend aus Nicht-Kalium-Diuretika, Angiotensin-Converting-Enzym-Inhibitoren, nicht-steroidalen entzündungshemmenden Medikamenten, Heparin und Trimethoprim, ausgewählt ist.

**15.** Verfahren zur Bestimmung der Adsorption von Kaliumionen, umfassend das Nachweisen einer Adsorptionskapazität des Polymers nach einem der Ansprüche 1 bis 9 für Kaliumionen durch Ionenchromatographie.

## Revendications

**1.** Polymère comprenant des unités répétitives obtenues par polymérisation d'un monomère et d'un agent de réticulation, dans lequel le rapport molaire entre le monomère et l'agent de réticulation est compris entre 1:0,02 et 1:0. 20, et le monomère comprend un groupe acide et un groupe pKa-réducteur à côté du groupe acide, dans lequel le groupe acide est choisi dans le groupe consistant en un groupe acide sulfonique ($-SO_3^-$), un groupe acide sulfurique ($-OSO_3^-$), un groupe carboxylique ($-CO_2^-$), un groupe acide phosphonique ($-OPO_3^{2-}$), un groupe phosphate ($-OPO_3^{2-}$), et un groupe acide sulfamique ($-NHSO_3^-$) ; le groupe réducteur de pKa est choisi dans le groupe constitué des atomes de nitro, de cyano, de carbonyle, de trifluorométhyle et d'halogène ; et l'agent de réticulation a trois ou quatre sites de réaction.

**2.** Polymère selon la revendication 1, dans lequel le groupe acide est le groupe carboxylique et le groupe réducteur de pKa est le fluor.

**3.** Polymère selon la revendication 1, dans lequel les sites de réaction sont des groupes alcényles libres.

**4.** Polymère selon la revendication 1, dans lequel l'agent de réticulation comprend au moins l'un des éléments suivants : isocyanurate de triallyle, 1,2,4-trivinylcyclohexane, tétravinylsilane, triméthacrylate de triméthylolpropane, cyanurate de triallyle ou triallylcarbinol.

**5.** Polymère selon la revendication 1, dans lequel le polymère est au moins un polymère choisi dans le groupe constitué

par le polymère d'acide sulfonique de polyvinyle, le polymère d'acide sulfamique de polyvinyle, le copolymère d'acide sulfamique de vinyle et d'acide sulfurique de vinyle, le polymère d'acide phosphonique d'amino-phosphate de polyvinyle, polymère de N-(bisphosphonate éthyl) polyvinylamine, polymère d'acide poly($\alpha$-fluoroacrylique), copolymère d'acide vinylphosphonique et d'acide acrylique, copolymère d'acide vinylphosphonique et d'acide $\alpha$-fluoroacrylique, polymère d'acide polyvinylsulfurique et polymère d'acide polyvinylsulfamique réticulé.

6. Polymère ayant une structure représentée par la formule (I) ou étant un sel de la structure représentée par la formule (I) :

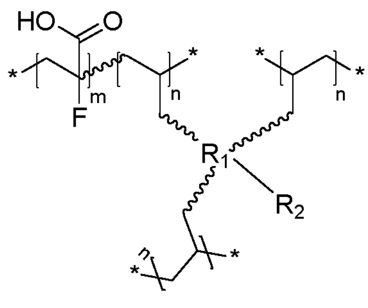

(I),

dans laquelle $R_1$ est

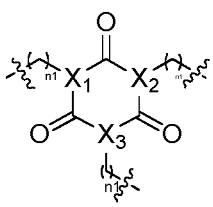
,
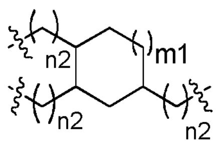
,
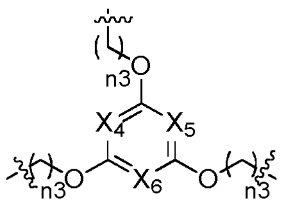
,

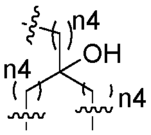
,
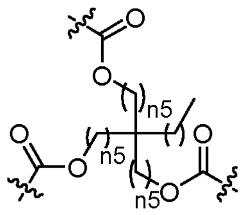
ou
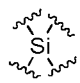
;

lorsque $R_1$ contient trois sites de liaison, $R_2$ n'est pas présent ; et lorsque $R_1$ contient quatre sites de liaison, R2 est connecté à l'un des quatre sites de liaison de $R_1$ et $R_2$ est

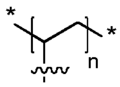
,

m est compris entre 0,80 et 0,98, n est compris entre 0,02 et 0,20, et m+n=1 ;
n1, n2, n3, n4 et n5 sont chacun indépendamment choisi parmi 0, 1, 2, 3, 4, 5, 6 ou 7 ;
$X_1$, $X_2$, $X_3$, $X_4$, $X_5$ et $X_6$ sont chacun indépendamment un atome de carbone ou un atome d'azote ;
m1 est 0 ou 1 ; et
* représente un site de liaison.

7. Polymère selon la revendication 6, dans lequel $R_1$ est

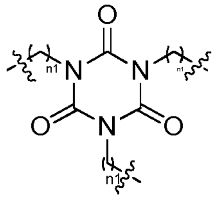

,

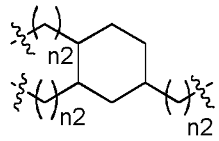, 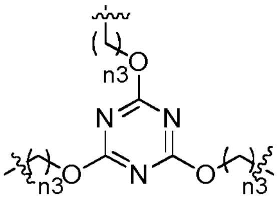, 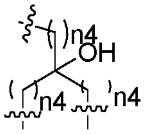, 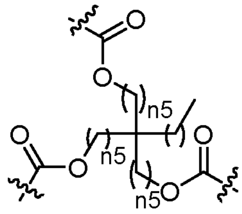 ou 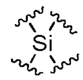.

**8.** Polymère selon la revendication 6, dans lequel $R_1$ est

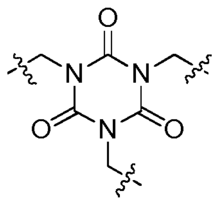

,

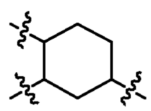, 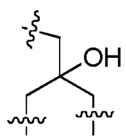, 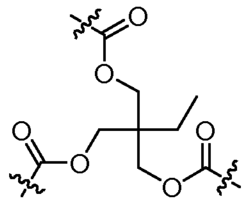 ou 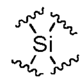.

**9.** Polymère présentant l'une des structures suivantes ou étant un sel de l'une des structures suivantes :

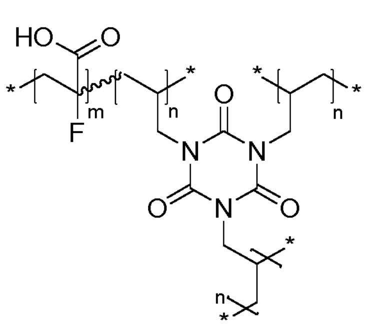, 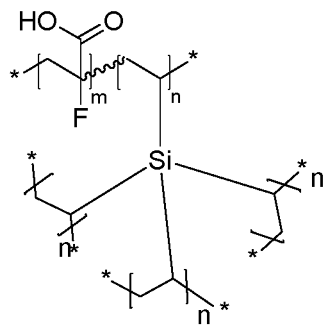 ou 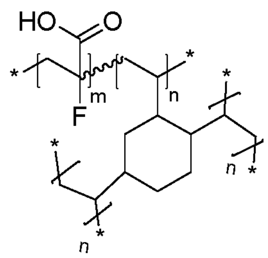,

dans lesquelles m est compris entre 0,80 et 0,98, n est compris entre 0,02 et 0,20, et m+n=1.

**10.** Composition pharmaceutique, comprenant le polymère selon l'une des revendications 1 à 9, et un excipient pharmaceutiquement acceptable.

**11.** Polymère selon l'une des revendications 1 à 9 ou composition selon la revendication 10 pour utilisation dans l'adsorption du potassium chez un sujet.

**12.** Polymère selon l'une quelconque des revendications 1 à 9 ou composition selon la revendication 10 pour utilisation

dans la prévention ou le traitement de l’hyperkaliémie.

**13.** Polymère selon l’une quelconque des revendications 1 à 9 ou composition selon la revendication 10 pour l’utilisation selon la revendication 12, dans lequel l’hyperkaliémie est causée par l’administration d’un médicament qui provoque une rétention de potassium.

**14.** Polymère selon l’une des revendications 1 à 9 ou composition selon la revendication 10 pour l’utilisation selon la revendication 13, dans lequel le médicament qui provoque une rétention de potassium est choisi dans le groupe constitué par les diurétiques non potassiques, les inhibiteurs de l’enzyme de conversion de l’angiotensine, les médicaments anti-inflammatoires non stéroïdiens, l’héparine et le triméthoprime.

**15.** Méthode de détermination de l’adsorption des ions potassium, comprenant : la détection d’une capacité d’adsorption du polymère selon l’une des revendications 1 à 9 pour les ions potassium par chromatographie ionique.

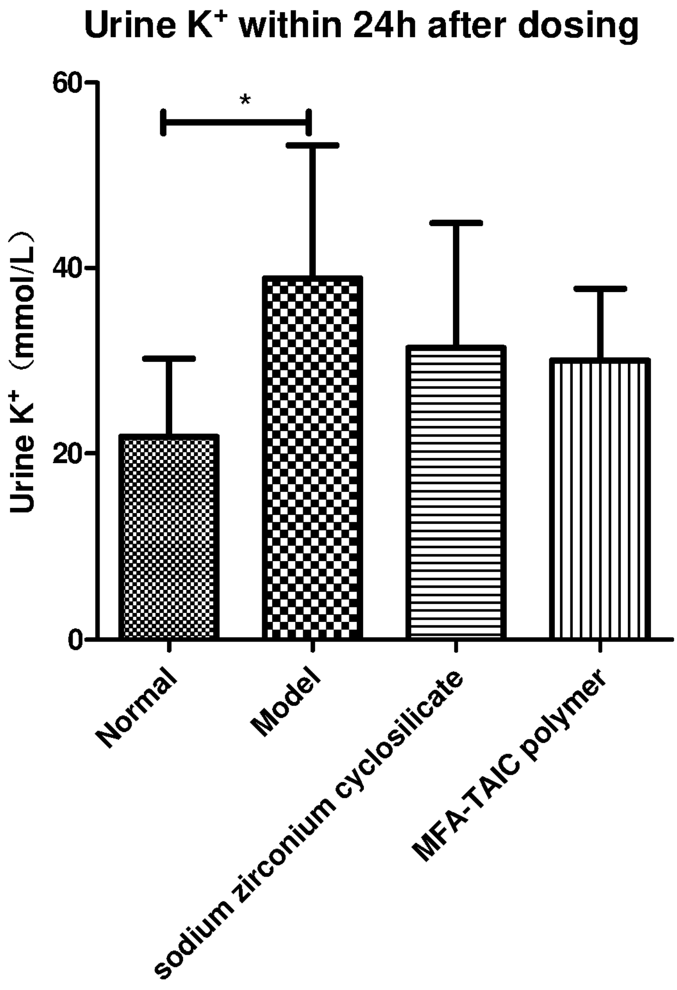
Urine K+ within 24h after dosing
Urine K+（mmol/L）
60
40
20
0
*
Normal
Model
sodium zirconium cyclosilicate
MFA-TAIC polymer

FIG. 1

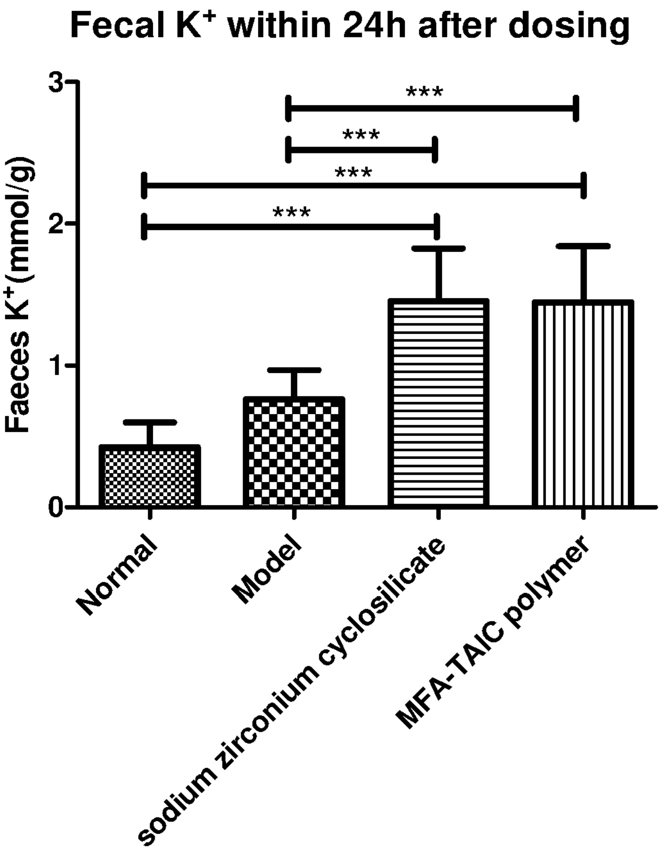
Fecal K+ within 24h after dosing
Faeces K+(mmol/g)
0
1
2
3
***
***
***
***
Normal
Model
sodium zirconium cyclosilicate
MFA-TAIC polymer

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2011236340 A1
- WO 2014015240 A1
- US 5112993 A
- EP 415214 A
- US 4427794 A


### Non-patent literature cited in the description


- **GASSENETAL.** *J. Fluorine Chemistry,* 1991, vol. 55, 149-162
- **KF PITTMAN ; C.U., M. UEDA et al.** *Macromolecules,* 1980, vol. 13 (5), 1031-1036
- **BOGACHEVETAL.** *Zhurnal Organisheskoi Khimii,* 1986, vol. 22 (12), 2578-83